(19)

Europäisches Patentamt
European Patent Office
Office européen des brevets

(11) **EP 4 289 442 A1**

(12) **EUROPEAN PATENT APPLICATION**
published in accordance with Art. 153(4) EPC

(43) Date of publication:
**13.12.2023 Bulletin 2023/50**

(21) Application number: **21947755.1**

(22) Date of filing: **20.07.2021**

(51) International Patent Classification (IPC):
*A61K 38/38* (2006.01)   *A61K 9/06* (2006.01)
*A61K 9/08* (2006.01)   *A61P 27/02* (2006.01)
*A61P 17/02* (2006.01)

(86) International application number:
**PCT/CN2021/107365**

(87) International publication number:
**WO 2023/272813 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2021   CN 202110722585**

(71) Applicants:
• **Huatong Forturn (China) Co., Ltd**
  **Beijing 100125 (CN)**
• **Beijing Bio-Fortune, Ltd.**
  **Beijing 102600 (CN)**
• **Huatong Forturn Biopharmaceutical (Shandong) Co., Ltd**
  **Tai'an, Shandong 271000 (CN)**

(72) Inventors:
• **FU, Yan**
  **Beijing 100125 (CN)**
• **LIU, Dianxin**
  **Beijing 100125 (CN)**
• **YANG, Xiaonan**
  **Beijing 100125 (CN)**
• **FU, Yusong**
  **Beijing 100125 (CN)**
• **HAN, Fang**
  **Beijing 100125 (CN)**
• **SONG, Liguang**
  **Beijing 100125 (CN)**
• **YANG, Jingsheng**
  **Beijing 100125 (CN)**
• **CHEN, Ying**
  **Beijing 100125 (CN)**

(74) Representative: **Huang, Liwei**
  **Cäcilienstraße 12**
  **40597 Düsseldorf (DE)**

(54) **TEMPERATURE SENSITIVE GEL DAMAGE REPAIR FORMULATION AND APPLICATION THEREOF**

(57)   The present invention belongs to the technical field of biomedical formulations. Provided are a temperature sensitive gel damage repair formulation and an application thereof. The formulation comprises 0.01-5 mg/mL of rHSA/EGF, 1.9-2.6% of glycine, 3.9-6.2% of poloxamer 188, 16.3-18.2% of poloxamer 407, and 0.5-100 mM of a PB buffer. The rHSA/EGF serves as an active ingredient, improvement of the repair of cornea and conjunctiva damage and the prevention, alleviation, and treatment of keratoconjunctivitis sicca are facilitated, and skin cell growth and damaged skin repair are also induced; an excipient can ensure structural stability of an active ingredient and preserve drug activity, while also causing the formulation to maintain an aqueous dosage from at 0-35 °C, and be gel-like at 35 °C or above, allowing the present formulation to remain at a drug application site for a longer period of time and making slow release of an active ingredient easier, and consequently improving the utilization of an active ingredient, shortening the duration of medication, and improving repair speed.

FIG. 1

EP 4 289 442 A1

## Description

This application claims priority to Chinese Patent Application No. 202110722585.8, filed to the CNIPA on June 29, 2021 and entitled "Temperature Sensitive Gel Damage Repair Formulation and Application Thereof, which is incorporated herein by reference in its entirety.

## Technical Field

The present disclosure belongs to the technical field of biomedical formulations, and particularly relates to a temperature sensitive gel damage repair formulation and an application thereof.

## Background

Dry eye syndrome may be referred to as keratoconjunctivitis xerosis (KCS) or xerophthalmia, is a disease of eye discomfort and visual dysfunction caused by tear film instability and ocular surface damage due to the abnormalities in the quantity or quality and hydrodynamics of tears, and may lead to cornea epithelium damage after long-term development. Common symptoms include ocular dryness, foreign body sensation, ocular dryness-caused syndromes, the density of goblet cells in patients' conjunctiva decreases, the ratio of nuclear to plasma increases, epithelial cells become squamous, corneal epithelium becomes conjunctival, and is damaged, so it is urgent to repair the cornea and conjunctiva. Keratoconjunctivitis sicca is now beginning to rejuvenate, i.e., there is an urgent need for innovative drugs for keratoconjunctivitis sicca prevention and clinical treatment in young people due to the use of computers and cell phones, which form a large number of actual clinical needs to be prevented or further subject to drug intervention and clinical treatment. The surface of the normal human eye continuously secretes a "protective film" formed by tears, also known as the tear film. The tear film is like a "hydration mask" and locks in moisture, forming a smooth tear film covering the surface of the eye, avoiding damage to the cornea during blinking. Tears contain various lysozymes and antibodies that kill microorganisms, protect ocular surface from damage, and remove foreign particles. Artificial tears, which may be eye drops, a care solution or may be referred to as a lubricating solution, are ophthalmic care solutions consisting of a series of functions for protecting the conjunctiva, cornea, lubrication, and water retention.

Damage, trauma, abrasions, and scratches of the outer skin of the human body are often occurring among the group of military, athletes, students, etc. Rapid repair and healing of damaged epidermis is desirable and necessary to ensure that the normal life of the human body. This does not seem to be a big problem, but it is very time-consuming and encumbered with considerable inconvenience to self-heal to return to normal life, which is an urgent need in the clinic for the emergent need of special effect products to become available as soon as possible.

Human serum albumin (HSA) is a soluble monomeric protein that makes up half of the total amount of protein in the blood. Albumin is the major component in blood, containing 50 grams per liter of blood in humans, and has a half-life of at least 20 days. Albumin, which is the most critical matrix (carrier) in human blood, can carry and deliver fatty acids, steroids and hormone molecules, particularly small molecule drugs, polypeptides, large molecule proteins, antibodies, etc., and continuously circulate in the blood. The stable inert nature of human serum albumin is an important factor in maintaining blood pressure. Human serum albumin is a globular non-glycosylated, large protein with a molecular weight of 65 kilodaltons and a mature peptide of 585 amino acids. Albumins currently used in the clinic are all extracted from human plasma. In pharmaceutical formulation compositions, albumin is also commonly used as a stabilizer for drugs, especially vaccines and biopharmaceuticals. By virtue of the excellent stability and inertness characteristics of albumin in blood, it is the practice of the present inventors to fuse proteins with very short half-lives to albumin genes via genetic engineering techniques, to clone and construct engineered strains of recombinant gene expression cells or engineered strains of gene expression yeast in foreign gene expression systems, to obtain soluble fusion proteins. A method for recombinant expression of albumin (rHSA) using microorganisms has been disclosed in Chinese granted patent ZL2004010057313.7 invented by by Yu Zailin and Fu Yan. After the therapeutic protein forms a fusion protein with albumin, the fusion protein would have the great advantage that it would be resistant to in vivo enzymolysis, can result in greatly improved longevity of the therapeutic protein in vivo and in vitro, can also be used at higher doses, or at lower doses compared to the clinically administered dose of the unfused monomeric therapeutic protein, and can also achieve comparable or better clinical efficacy (see, e.g., Yu Zailin and Fu Yan: A better biological innovation drug-recombinant human albumin fusion proteins with long-lasting biological effects, Chinese Medicinal Biotechnology, 2017).

Human Epidermal Growth Factor (hEGF) is a biologically active protein composed of 53 amino acids secreted by the human body, is widely distributed in body fluids such as blood, saliva, and urine, has a wide range of biological activities, and promotes the division of epidermal cells, accelerates cellular metabolism, and allows new epidermal cells to rapidly replace aged cells. The blood half-life of EGF is only around 1 hour, and its half-life is extremely short, resulting in a drug action time which, although slightly improved, still requires frequent administration for good clinical efficacy. To this end, scientists use genetic engineering techniques to make epidermal growth factor long-lasting, fuse EGF with human serum albumin gene, construct a genetically engineered strain of Pichia pastoris, and use an exogenous expression production system to prepare recombinant human serum albumin/epidermal growth factor fusion protein [ZL200710057571.9, WO2009/043277A1, US8, 603,973B2]. Accordingly, a number of different bio-innovative drugs have been developed to allow recombinant serum albumin fusion proteins to be administered by injection, for example: recombinant human serum albumin/granulocyte stimulating factor fusion protein for injection, recombinant human serum albumin/interferon α2a fusion protein for injection, recombinant human serum albumin/interferon α2b fusion protein for injection, recombinant human serum albumin/erythropoietin fusion protein for injection, etc. However, the fusion protein injection ensures the superior efficacy of the fusion protein, however, external administration is not applicable, such as keratoconjunctivitis sicca, keratitis and cornea damage (collectively referred to as cornea and conjunctiva damage). Post-cornea surgery repair is also a common clinical condition requiring drug intervention, and skin epidermal damage administration is not suitable for administration by injection and is suitable for external administration. However, for traditional external administration of, such as eye drops and external skin sprays for the eye which are both in the aqueous-like form, only a very small part of the drug can be absorbed by the local application after the administration, and most of the drug flows to the non-application site by gravity, which results in not only loss of drug efficacy but also serious waste of the drug, which makes it difficult to evaluate the efficacy of the drug and to determine the clinical treatment means.

**Summary**

In view of this, the present disclosure aims to provide a temperature sensitive gel damage repair formulation and an application thereof. The formulation can not only preserve the biological activity of recombinant human serum albumin/epidermal growth factor fusion protein (rHSA/EGF) well, but also form a gel at a drug application site so as to remain at the drug application site for a longer period of time, and make slow release of active fusion protein in the formulation easier so as to achieve a longer half-life, thereby improving the treatment effect of the formulation.

The present disclosure provides a temperature sensitive gel damage repair formulation, including components at the following concentrations: 0.01-5 mg/mL of recombinant human serum albumin/epidermal growth factor fusion protein, 1.9-2.6% m/m glycine, 3.9-6.2% m/m poloxamer 188, 16.3-18.2% m/m poloxamer 407, and 0.5-100 mM of phosphate buffer (PB).

Preferably, the temperature sensitive gel damage repair formulation includes components at the following concentrations: 0.01-5 mg/mL of the recombinant human serum albumin/epidermal growth factor fusion protein, 1.9-2.1% m/m glycine, 5.8-6.2% m/m poloxamer 188, 16.3-16.7% m/m poloxamer 407, and 0.5-100 mM of the PB.

Preferably, the temperature sensitive gel damage repair formulation includes components at the following concentrations: 0.2 mg/mL of the recombinant human serum albumin/epidermal growth factor fusion protein, 2.0% m/m glycine, 6.0% m/m poloxamer 188, 16.5% m/m poloxamer 407, and 10 mM of the PB.

Preferably, the temperature sensitive gel damage repair formulation includes components at the following concentrations: 0.01 to 5 mg/mL of the recombinant human serum albumin/epidermal growth factor fusion protein, 1.8-2.2% m/m glycine, 5.8-6.2% m/m poloxamer 188, 17.8-18.2% m/m poloxamer 407, and 0.5 to 100 mM of the PB.

Preferably, the temperature sensitive gel damage repair formulation includes components at the following concentrations: 0.2 mg/mL of the recombinant human serum albumin/epidermal growth factor fusion protein, 2.0% m/m glycine, 6.0% m/m poloxamer 188, 18% m/m poloxamer 407, and 10 mM of the PB.

Preferably, the temperature sensitive gel damage repair formulation includes components at the following concentrations: 0.01-5 mg/mL of the recombinant human serum albumin/epidermal growth factor fusion protein, 2.0-2.5% m/m glycine, 4.9-5.1% m/m poloxamer 188, 17.8-18.2% m/m poloxamer 407, and 0.5-100 mM of the PB.

Preferably, the temperature sensitive gel damage repair formulation includes components at the following concentrations: 0.2 mg/mL of the recombinant human serum albumin/epidermal growth factor fusion protein, 2.0% m/m glycine, 5.0% m/m poloxamer 188, 18% m/m poloxamer 407, and 10 mM of the PB.

The present disclosure provides the temperature sensitive gel damage repair formulation including temperature sensitive gel eye drops and temperature sensitive gel external spray.

The present disclosure provides an application of the temperature sensitive gel damage repair formulation in preparing a drug for repairing cornea and/or conjunctiva damage.

The present disclosure provides an application of the temperature sensitive gel damage repair formulation in preparing a drug in preventing and/or treating keratitis.

The present disclosure provides an application of the temperature sensitive gel damage repair formulation in preparing a drug for preventing, alleviating or treating keratoconjunctivitis sicca.

The present disclosure provides an application of the temperature sensitive gel damage repair formulation in preparing a drug for treating skin epidermal trauma, abrasions or ulcers.

The present disclosure provides an application of the temperature sensitive gel damage repair formulation in repairing cornea and/or conjunctiva damage.

The present disclosure provides an application of the temperature sensitive gel damage repair formulation in treating keratitis and/or keratoconjunctivitis sicca.

The present disclosure provides an application of the temperature sensitive gel damage repair formulation in treating skin epidermal trauma, abrasions or ulcers.

The present disclosure provides the temperature sensitive gel damage repair formulation, including the components at the following concentrations: 0.01-5 mg/mL of the recombinant human serum albumin/epidermal growth factor fusion protein, 1.9-2.6% m/m glycine, 3.9-6.2% m/m poloxamer 188, 16.3-18.2% m/m poloxamer 407, and 0.5-100 mM of the PB. The temperature sensitive gel damage repair formulation provided by the present disclosure takes the recombinant human serum albumin/epidermal growth factor fusion protein as an active ingredient, and may enter aqueous humor via eye drop, improvement of the repair of cornea and conjunctiva damage and the prevention, alleviation, and treatment of keratoconjunctivitis sicca are facilitated; the temperature sensitive gel damage repair formulation may also enter the skin layer via percutaneous absorption for inducing skin cell growth and damaged skin repair; at the same time, the temperature sensitive gel damage repair formulation further includes excipients such as glycine, poloxamer 188, poloxamer 407 and the PB, which not only can guarantee the structural stability of the active ingredient, thus guaranteeing the drug activity of the active ingredient, but also can regulate appropriate osmotic pressure for providing guarantee for ophthalmic drug delivery; in addition, the excipients enable the formulation to maintain a clear and transparent aqueous dosage form at 0-35 °C, and be gel-like at 35 °C or above; compared with a conventional non-temperature-sensitive formulation, the formulation provided by the present disclosure can remain at a drug application site for a longer period of time, the slow release of an active protein molecule in the formulation is easier so as to achieve a longer half-life, thereby ensuring the exertion of the pharmaceutical effect, increasing the utilization of the active ingredient, shortening the duration of medication, and increasing the repair speed. At the same time, the formulation is liquid at 2-35 °C, which is beneficial to production preparation; the formulation forms a gel when making contact with the human epidermis or eyeballs. The formulation is safe, non-irritating, and non-toxic to the human body, and if the formulation is made into a daily disposable package, it will be more convenient for a patient for self-medication.

**Brief Description of Figures**

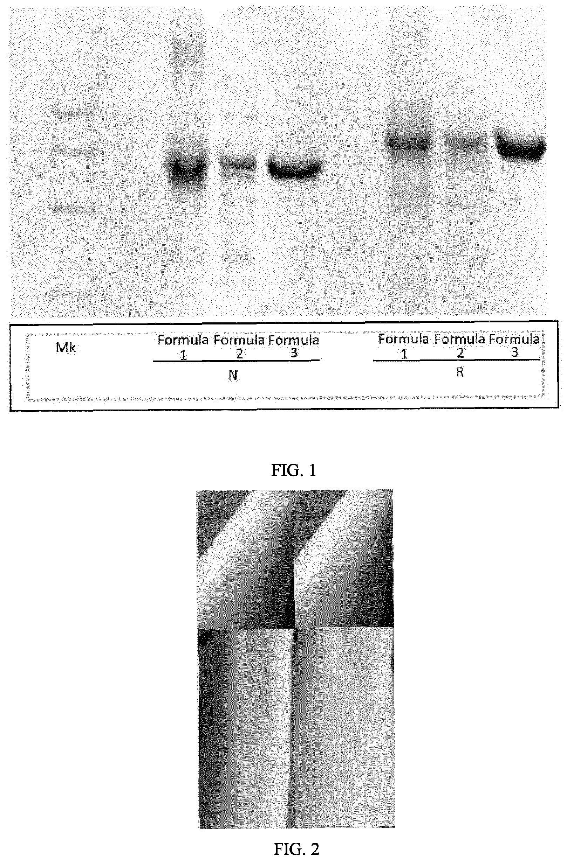

FIG. 1 shows the results of stability testing of rHSA/EGF fusion protein in the formulation formulas provided by the present disclosure, wherein from left to right, SSDS-PAGE gel electrophoretogram band 1 is standard molecular weight MK; band 2 is blank, bands 3-5 are protein electrophoresis results of formulations formulas 1 to 3 under non-reducing conditions; band 6 is blank; bands 7-9 are electrophoresis results of formulation formulas 1 to 3 under reducing conditions;

FIG. 2 shows that recombinant human serum albumin/epidermal growth factor fusion protein temperature sensitive gel eye drops and recombinant human serum albumin/epidermal growth factor fusion protein eye drops (spray) are simultaneously applied to healthy volunteers (left: 0 min after administration; right: 15 min after administration; the upper two figures are from male volunteers, and the lower two figures are from female volunteers), no epidermal irritation, allergy, or damage was found after single or multiple times of application, the temperature sensitive gel eye drops form a gel due to the epidermal temperature after contacting the skin, which can last for at least 15 min; whereas the non-temperature-sensitive non-gel-like eye drops (or sprays) do not aggregate and stay on the skin surface and leave no trace after absorption;

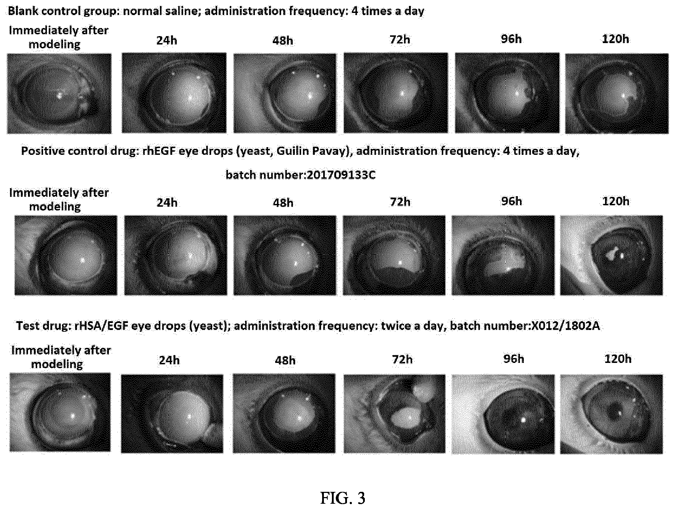

FIG. 3 shows the results of pharmacodynamic study of blank control (normal saline), positive control (rhEGF eye drops commodity ) and rHSA/EGF temperature sensitive gel eye drops in a cornea damage animal model established by scraping all cornea epithelium in the right eyes of rabbits; immediately after modeling, and at 24 h, 48 h, 72 h, 96 h and 120 h after the first administration, corneal pictures are observed and collected by a slit-lamp microscope, the area ($mm^2$) of corneal fluorescence staining are measured, and the area differences between different groups are compared;

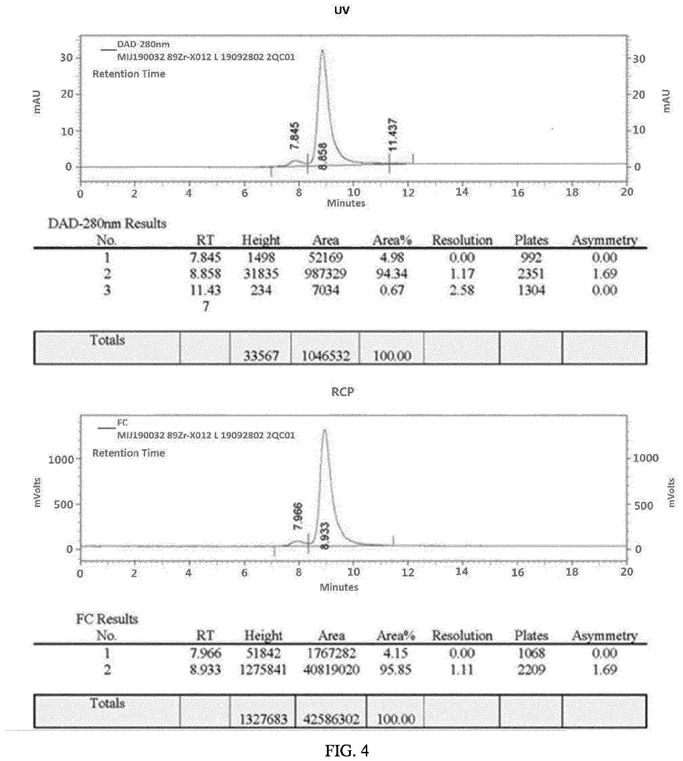

FIG. 4 is the results of SEC-HLPC analysis of rHSA/EGF integrity before and after radiolabeling;

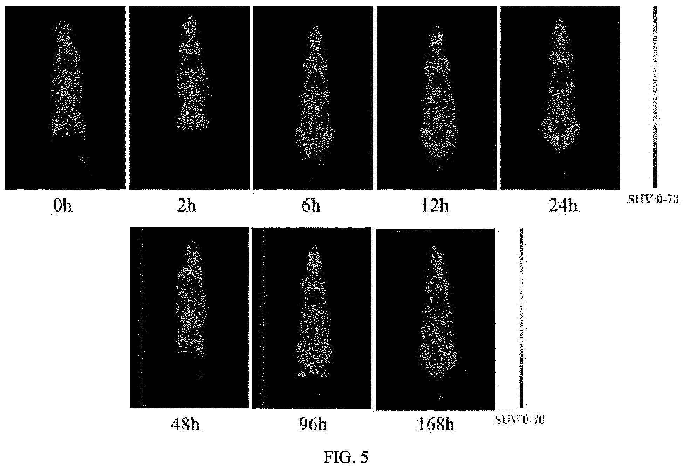

FIG. 5 shows tissue distribution study and pharmacokinetic observation of 89Zr-recombinant human serum albumin/epidermal growth factor fusion protein temperature sensitive gel eye drops being administered to New Zealand Rabbits for a single time. The PET/CT scanning coronal layered graphs at different time points show the scanning results of 0 h, 2 h, 6 h, 12 h, 24 h, 48 h, 96 h and 168 h, respectively, showing the distribution of animal tissues and the changes of pharmacokinetics; and

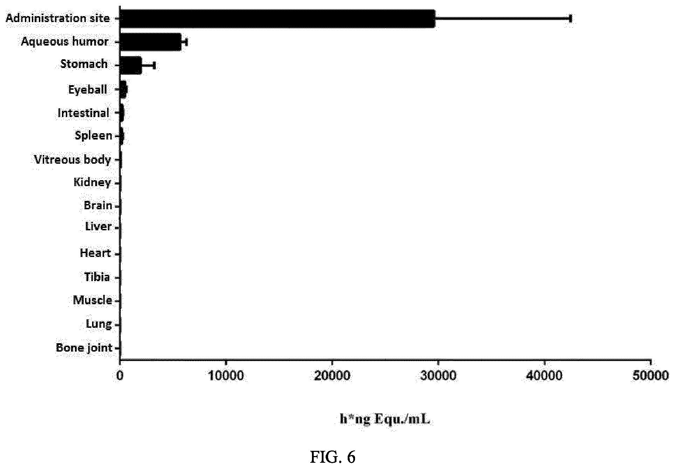

FIG. 6 is a distribution diagram of radioactive counts for each tissue, showing the metabolic profile of the test drug eye drops being administered to eyeballs of the white rabbits.

**Detailed Description**

The present disclosure provides a temperature sensitive gel damage repair formulation, which includes the components at the following concentrations: 0.01-5 mg/mL of recombinant human serum albumin/epidermal growth factor fusion protein, 1.9-2.6% m/m glycine, 3.9-6.2% m/m poloxamer 188, 16.3-18.2% m/m poloxamer 407, and 0.5-100 mM of phosphate buffer (PB).

In the present disclosure, in the temperature sensitive gel damage repair formulation, the recombinant human serum albumin/epidermal growth factor fusion protein (rHSA/EGF) serves as an active ingredient. The source of the recombinant human serum albumin/epidermal growth factor fusion protein is not particularly limited in the present disclosure, and sources of human serum albumin/epidermal growth factor fusion protein known in the art may be adopted. In examples of the present disclosure, the nucleotide sequences of the recombinant human serum albumin/epidermal growth factor fusion protein and preparation methods are disclosed in patent documents with the application numbers of ZL200710057571.9, WO2009/043277A1 and US8,603,973B2. Distribution of active ingredients and pharmacokinetic trials show that the radioactivity concentrations of the tissue distribution of $^{89}$Zr-recombinant human serum albumin/epidermal growth factor fusion protein rank from high to low as administration site > aqueous humor > stomach > eyeball > intestine > spleen > vitreous body > kidney > brain > liver > heart > tibia > muscle > lung > bone joint, which indicates that the recombinant human serum albumin/epidermal growth factor fusion protein is distributed primarily in the administration site after being administered, and can enter the body through the eyes to the cornea damage site.

In the present disclosure, the temperature sensitive gel damage repair formulation further includes excipients. The excipients include glycine, poloxamer 188, poloxamer 407, and the PB. Experiments show that the excipients, after stringent concentration screening as described above, have the following effects: 1) the stability of the active ingredient rHSA/EGF can be enhanced to preserve the original activity of the rHSA/EGF, 2) the rHSA/EGF in the matrix is more susceptible to steric hindrance effects during release at the application site, thus by adjusting components added to the formulation formula, and the dosage and ratio of the added components, the sustained release of the rHSA/EGF is more effective; 3) the added excipients have no irritating and toxic and side effects on the human body, in particular on the eyes; 4) the use of excipient components should meet pharmacopoeia standards without deleterious reactions and effects on the human body and the application site, as well as on rHSA/EGF activity; 5) the type and proportion of the excipients can ensure that the formulation is capable of maintaining a clear and transparent aqueous dosage form under the condition of 0-35 °C, and forming a gel at the administration site at 35 °C or above, and remaining at the administration site for a longer period of time to allow slow release of rHSA/EGF, so as to give fully play to the drug effect, and improve the reparative and therapeutic effect of the formulation.

In the present disclosure, the temperature sensitive gel damage repair formulation is obtained by optimizing a non-temperature-sensitive damage repair formulation. Previous studies show that, glycerol has the effect of stabilizing the protein, is also a water-retaining agent, and has the function and effect of adjusting the osmotic pressure; but the prepared formulation has high fluidity, on the basis of containing the glycerol, even if the added CMC has the effect of increasing the viscosity, can stabilize the eye drops and reduce the local fluidity of the eye drops, but the stability of protein does not meet the requirements. In view of this, poloxamer added on the basis of glycine has good viscosity, can form a gel, can reduce the local fluidity of eye drops, and meets the basic requirements of fusion protein stability. On this basis, different temperature sensitive gel formulations are prepared by adjusting and controlling the proportions of glycine, poloxamer 188 and poloxamer 407.

In the present disclosure, the temperature sensitive gel damage repair formulation preferably includes components at the following concentrations: 0.01-5 mg/mL of the recombinant human serum albumin/epidermal growth factor fusion protein, 1.9-2.1% m/m glycine, 5.8-6.2% m/m poloxamer 188, 16.3-16.7% m/m poloxamer 407, and 0.5-100 mM of the PB, and more preferably includes components at the following concentrations: 0.2 mg/mL of the recombinant human serum albumin/epidermal growth factor fusion protein, 2.0% m/m glycine, 6.0% m/m poloxamer 188, 16.5% m/m poloxamer 407, and 10 mM of the PB. The temperature at which the formulation forms a gel is greater than or equal to 35.1 °C, and the increase in glycine content does not affect protein stability and biological activity.

In the present disclosure, the temperature sensitive gel damage repair formulation preferably includes components at the following concentrations: 0.01-5 mg/mL of the recombinant human serum albumin/epidermal growth factor fusion protein, 1.9-2.1% m/m glycine, 5.8-6.2% m/m poloxamer 188, 17.8-18.2% m/m poloxamer 407, and 10 mM of the PB, and more preferably includes components at the following concentrations: 0.2 mg/mL of the recombinant human serum albumin/epidermal growth factor fusion protein, 2.0% m/m glycine, 6.0% m/m poloxamer 188, 18% m/m poloxamer 407 and 10 mM of the PB. The temperature at which the formulation forms a gel is greater than or equal to 33.5 °C.

In the present disclosure, the temperature sensitive gel damage repair formulation preferably includes components at the following concentrations: 0.01-5 mg/mL of the recombinant human serum albumin/epidermal growth factor fusion protein, 2.0-2.5% m/m glycine, 4.9-5.1% m/m poloxamer 188, 17.8-18.2% m/m poloxamer 407, and 0.5-100 mM of the PB, and more preferably includes components at the following concentrations: 0.2 mg/mL of the recombinant human serum albumin/epidermal growth factor fusion protein, 2.0% m/m glycine, 5.0% m/m poloxamer 188, 18% m/m poloxamer 407 and 10 mM of the PB.

In the present disclosure, the temperature sensitive gel damage repair formulation preferably includes temperature sensitive gel eye drops and temperature sensitive gel external spray.

In the present disclosure, a preparation method of the temperature sensitive gel damage repair formulation preferably includes mixing the recombinant human serum albumin/epidermal growth factor fusion protein, glycine, poloxamer 188, poloxamer 407 and the PB. The glycine is prepared into a stock solution and then sterilized by filtration through a 0.2 $\mu$M filter membrane for later use. The poloxamer 188 and the poloxamer 407 are preferably prepared into 10-200 times stock solutions, and autoclaved at 120-125 °C for 20 minutes at the pressure of 0.1 MPa, and then cooled for later use. Preparation methods of the temperature sensitive gel eye drops and the temperature sensitive gel external spray are not particularly limited in the present disclosure, and preparation methods of the eye drops and external spray known in the art may be used.

The present disclosure provides an application of the temperature sensitive gel damage repair formulation in preparing a drug for repairing cornea and/or conjunctiva damage.

The present disclosure provides an application of the temperature sensitive gel damage repair formulation in preparing a drug for preventing and/or treating keratitis.

The present disclosure provides an application of the temperature sensitive gel damage repair formulation in preparing a drug for preventing, alleviating or treating keratoconjunctivitis sicca.

The present disclosure provides an application of the temperature sensitive gel damage repair formulation in preparing a drug for treating skin epidermal trauma, abrasions or ulcers.

The temperature sensitive gel damage repair formulation and the application thereof provided by the present disclosure will be described in detail below with reference to examples, but the examples should not be construed as limiting the scope of the present disclosure.

**Comparative Example 1**

1. The specific component content of a formulation formula 1 containing rHSA/EGF is listed in Table 1.

Table 1 Formulation formula 1 containing rHSA/EGF

| Serial Number | Name | Amount | Use |
| --- | --- | --- | --- |
| 1 | rHSA/EGF | 0.2 mg/mL | Drug substance |
| 2 | Glycerol | 2.4% | Osmotic pressure regulation |
| 3 | 100 mM PB | To final concentration of 10 mM | Buffer |

Solutions were prepared according to the formula of Table 1 to prepare 10 bottles of samples (4 ml/bottle) for later use.

2. The prepared formulation was subjected to accelerated testing to analyze the stability of formulation

1) the container and packaging material and manner of packaging of the test article used for the drug should be as consistent as possible with those of the marketed product;

2) in drug stability test, drug analysis methods with strong specificity, accuracy, precision and sensitivity should be adopted to ensure the reliability of drug stability results, and homogenous physicochemical controls and homogenous bioactive standards can be prepared when necessary;

3) for products subjected to accelerated stability testing, the preparation amount of each batch of solution should be greater than 8 times of the complete inspection amount at one time;

4) the formulation is placed at the temperature of 25 °C $\pm$ 2 °C and the relative humidity of 25% $\pm$ 2% for a specified period of time or 6 months (a saturated solution of $CH_3COOK\cdot1.5H_2O$ can be used); the equipment used should be capable of controlling the temperature to be $\pm$ 2 °C and the relative humidity to be $\pm$ 5% and monitoring the actual temperature and humidity;

5) sampling according to the specified inspection time shall be based on the date of the first inspection. Sampling shall not be carried out in advance within 3 months of accelerated test, but sampling may be carried out within 3 days after the specified sampling time; the sampling time after 3 months (inclusive) may be the specified time $\pm$ 3 days.

3. The prepared formulations were subjected to biological activity assay

The specific assay method was carried out in accordance with the General Principle 3528 of the Chinese Pharmacopoeia 2015 Edition Volume III.

4. The prepared formulations were subjected to protein electrophoresis detection

For the osmotic pressure, protein content and SDS-PAGE electrophoresis detection method of the formulations, please refer to General Principles 0632, 0731 and 0541 of relevant verification items in the Chinese Pharmacopoeia 2015 Edition Volume III. The osmotic pressure of each formulation formula was maintained at 300 ± 60 mOsmol/kg.

5. Detection of microbial limits is carried out in accordance with the General Principle 1101 of the Chinese Pharmacopoeia 2015 Edition Volume III. The finished products obtained by the formulas of the formulation all meet the requirements of sterility in the *Procedure for Preparation and Inspection* of this product.

The results are shown in FIG. 1 and Table 4. The results show that glycerol has the effect of stabilizing the protein, is also a water-retaining agent, and has the function and effect of adjusting the osmotic pressure. The stability of the fusion protein was subjected to accelerated observation in the formulation formula, and the obtained protein electrophoresis results are electrophoresis results of Formula 1 shown in FIG. 1.

**Comparative Example 2**

Formulation formula 2 containing rHSA/EGF

According to the analysis results of the formulation formula 1, the formulation formula with glycerin only does not meet the requirements on the formulation formula having a temperature-sensitive type and a gel type in terms of viscosity. To this end, carboxy methyl cellulose Na (CMC) was added to the formulation formula components, and the specific composition is shown in Table 2.

Table 2 Formulation formula 2 containing rHSA/EGF

| Component Serial Number | Name | Amount | Use |
|---|---|---|---|
| 1 | rHSA/EGF | 0.2 mg/mL | Drug substance |
| 2 | Glycerol | 2.4% | Osmotic pressure regulation |
| 3 | CMC | 0.1% | Viscosity regulation |
| 4 | 100 mM PB | To final concentration of 10 mM | Buffer |

10 bottles of samples (4 ml/bottle) were prepared. Accelerated stability observation was carried out for 14 days in an accelerated test chamber at a temperature of 25 °C and a humidity of 70% according to the method described in Comparative Example 1, and samples were taken at time nodes for appearance inspection, biological activity assay, protein electrophoresis detection and microbial limit detection. The results are shown in FIG. 1 and Table 4.

Even though the added CMC has a certain viscosity that stabilizes the eye drops and reduces the local fluidity of the eye drops on the basis of glycerol, the results of the fusion protein stability observation indicate that the protein stability does not meet the desired requirements compared with formulations containing only glycerol (see electrophoresis results of Formula 2 in FIG. 1).

**Example 1**

Formulation formula 3 containing rHSA/EGF

In view of the fact that the addition of glycerol in Comparative Example 1 or the simultaneous addition of glycerol and CMC in Comparative Example 2 did not significantly improve the stability of the fusion protein, glycine plus poloxamer were used instead as formulation excipients for protein stability and increasing viscosity of eye drops, as shown in Table 3 specifically.

Table 3 Formulation formula 3 containing rHSA/EGF

| Component Serial Number | Name | Amount | Use |
|---|---|---|---|
| 1 | rHSA/EGF | 0.2 mg/mL | Drug substance |
| 2 | Glycine | 2% | Stabilizer |
| 3 | Poloxamer 188 | 5% | Thickener |
| 4 | Poloxamer 407 | 18% | Thickener |
| 5 | 100 mM PB | To final concentration of 10 mM | Buffer |

10 bottles of samples (4 ml/bottle) were prepared. Accelerated stability observation was carried out for 14 days in an accelerated test chamber at a temperature of 25 °C and a humidity of 70% according to the method described in Comparative Example 1, and samples were taken at time nodes for appearance inspection, biological activity assay and protein electrophoresis detection.

The results are shown in Table 4.

Table 4 List of test results for three formulation formulas

| Example | Time | Appearance | Osmotic pressure | SDS-PAGE purity (%) | Bio-specific activity value (%) |
|---|---|---|---|---|---|
| Formulation formula 1 | 0 week | Clear aqueous dosage form, no visible precipitate | 280 | 100 | 100 |
| | 2 weeks | Clear aqueous dosage form, no visible precipitate | 281 | 96 | 96 |
| Formulation formula 2 | 0 week | Clear aqueous dosage form, no visible precipitate | 286 | 100 | 100 |
| | 2 weeks | Clear aqueous dosage form, slight precipitate | 289 | 78 | 91 |
| Formulation formula 3 | 0 week | Clear aqueous dosage form, no visible precipitate | 290 | 100 | 100 |
| | 2 weeks | Clear aqueous dosage form, no visible precipitate | 289 | 105 | 104 |

At the time of testing, the bio-specific activity value of the sample at day 0 was determined to be 100%, and the difference between the relative bio-specific activity values at different time points and day 0 was observed as a basis for calculation. Due to the addition of components affecting the integrity of the protein in the individual formulation formulas (formulation formula 2), the SDS-PAGE electrophoresis results showed that degradation occurred and the biological activity assay could not be demonstrated. This is because the degradation product contains monomers of EGF, and because of the short time, the bio-specific activity of protein fragments containing EGF does not appear to decrease significantly. This is because the EGF bioactivity of the monomer is much higher than that of the fusion protein (rhEGF bio-specific activity value: $5 \times 10^5$ IU/mg; rHSA/EGF bio-specific activity: $8 \times 10^4$ IU/mg; HSA molecular weight is 66 Kd; EGF molecular weight is 0.68 Kd according to the Chinese Pharmacopoeia 2015 Edition Volume III. There is a 6.2-fold difference in the specific activity value between the two since HSA has no epidermal growth factor activity).

Further, the results of Formula 3 show that the Poloxamer 188 and Poloxamer 407 added on the basis of glycine have good viscosity, can form a gel, and can reduce the local mobility of eye drops. Fusion protein stability observation also indicated that the stability of the protein was better compared with formulation formulas containing only glycerol, and reached the expected requirement of protecting the fusion protein. The results of the study indicate that formulation formula 3 meets the basic requirements of formulation formula on fusion protein stability.

**Comparative Example 3**

Formula 4 of temperature sensitive gel formulation

In order to further prepare a temperature sensitive gel formulation suitable for human body, formula 4 is provided (see Table 5) on the basis of formula 3, and by adjusting the amounts of poloxamer 188 and poloxamer 407, the goal that the eye drops are in a liquid state at the temperature of 0-35 °C, and are in a gel state (significantly reducing the fluidity of eye drops) at the temperature of above 35 °C is achieved.

Table 5 Temperature sensitive gel formulation

| Component Serial Number | Name | Amount | Use |
|---|---|---|---|
| 1 | rHSA/EGF | 0.2 mg/mL | Drug substance |
| 2 | Glycine | 2.5% | Stabilizer |
| 3 | Poloxamer 188 | 4% | Thickener |
| 4 | Poloxamer 407 | 19% | Thickener |
| 5 | 100 mM PB | To final concentration of 10 mM | Buffer |

10 bottles of samples (4 ml/bottle) were prepared. Accelerated stability observation was carried out for 14 days in an accelerated test chamber at a temperature of 25 °C and a humidity of 70% according to the method described in Comparative Example 1, and samples were taken at time nodes for appearance inspection, biological activity assay and protein electrophoresis detection. In addition, the prepared formulation was put into a 40 °C water bath, a thermometer was inserted into the sample, the fluidity of the eye drops was observed at all times, and the solidification temperature of the formulation formula was found as the temperature rose.

The results are shown in Table 7. The results show that the temperature sensitive gel formation temperature of this formulation formula is 39.2 °C. It was also determined that an increase in glycine content did not affect protein stability and biological activity.

## Example 2

Formula 5 of temperature sensitive gel formulation

In order to achieve the goal that the eye drops are in a liquid state at the temperature of 0-35 °C, and are in a gel state (significantly reducing the fluidity of eye drops) at the temperature of above 35 °C, the amounts of poloxamer 188 and poloxamer 407 was further adjusted on the basis of formulation formula 4, as shown in Table 6 specifically.

Table 6 Formula 5 of temperature sensitive gel formulation

| Component Serial Number | Name | Amount | Use |
|---|---|---|---|
| 1 | rHSA/EGF | 0.2 mg/mL | Drug substance |
| 2 | Glycine | 2% | Stabilizer |
| 3 | Poloxamer 188 | 6% | Thickener |
| 4 | Poloxamer 407 | 16.5% | Thickener |
| 5 | 100 mM PB | To final concentration of 10 mM | Buffer |

10 bottles of samples (4 ml/bottle) were prepared. Accelerated stability observation was carried out for 14 days in an accelerated test chamber at a temperature of 25 °C and a humidity of 70% according to the method described in Comparative Example 1, and samples were taken at time nodes for appearance inspection, biological activity assay and protein electrophoresis detection. In addition, the prepared formulation was put into a 40 °C water bath, a thermometer was inserted into the sample, the fluidity of the eye drops was observed at all times, and the solidification temperature of the formulation formula was found as the temperature rose. The flowability results for Formula 5 show that the temperature sensitive gel formation temperature of this formulation formula is 35.1 °C.

The results of the accelerated stability test observation are shown in Table 8.

## Example 3

Formula 6 of temperature sensitive gel formulation

In order to achieve the goal that the eye drops are in a liquid state at the temperature of 0-35 °C, and are in a gel state (no fluidity) at the temperature of above 35 °C, the amounts of poloxamer 188 and poloxamer 407 was further adjusted on the basis of formula 5, as shown in Table 7 specifically.

Table 7 Formula 6 of temperature sensitive gel formulation

| Component Serial Number | Name | Amount | Use |
|---|---|---|---|
| 1 | rHSA/EGF | 0.2 mg/mL | Drug substance |
| 2 | Glycine | 2% | Stabilizer |
| 3 | Poloxamer 188 | 6% | Thickener |
| 4 | Poloxamer 407 | 18% | Thickener |
| 5 | 100 mM PB | To final concentration of 10 mM | Buffer |

10 bottles of samples (4 ml/bottle) were prepared. Accelerated stability observation was carried out for 14 days in an accelerated test chamber at a temperature of 25 °C and a humidity of 70% according to the method described in Comparative Example 1, and samples were taken at time nodes for appearance inspection, biological activity assay and protein electrophoresis detection. In addition, the prepared formulation was put into a 40 °C water bath, a thermometer was inserted into the sample, the fluidity of the eye drops was observed at all times, and the solidification temperature of the formulation formula was found as the temperature rose. The results show that the temperature sensitive gel formation temperature of this formulation formula is 33.5 °C. The temperature sensitive gel formation temperature of formulation formula 4 is 39 °C, showing too high. Compared with the formulation formula 6, the formulation formula 5 can form a gel at 35 °C, is better than forming a gel at 33.5 °C as temperature sensitive gel eye drops, and is more resistant to remain non-gel-like at room temperature. Therefore, the formulation formula 5 is preferred to prepare temperature sensitive gel eye drops or external spray.

The results of the three formulas subjected to the Acceleration Stability Test observation are shown in Table 8.

Table 8 List of test results of the three formulas

| Table -8 | | | | | |
|---|---|---|---|---|---|
| Example | Time | Appearance | Osmotic pressure (300 ± 60 mOsmol/kg) | SDS-PAGE purity (%) | Bio-specific activity value (%) |
| Formulation formula 4 | 0 day | Clear aqueous dosage form, no visible precipitate | 280 | 100 | 100 |
| | one month | Clear aqueous dosage form, no visible precipitate | 287 | 96 | 104 |
| | two months | Clear aqueous dosage form, no visible precipitate | 290 | - | - |
| | three months | Clear aqueous dosage form, no visible precipitate | 281 | 96% | 96 |
| | Four months | Clear aqueous dosage form, no visible precipitate | 285 | - | - |
| | Five months | Clear aqueous dosage form, no visible precipitate | 292 | - | - |
| | Six months | Clear aqueous dosage form, no visible precipitate | 286 | 97 | 106 |
| Formulation formula 5 | 0 day | Clear aqueous dosage form, no visible precipitate | 297 | 100 | 100 |
| | one month | Clear aqueous dosage form, no visible precipitate | 286 | 97 | 110 |
| | two months | Clear aqueous dosage form, no visible precipitate | 289 | - | - |

(continued)

| Example | Time | Appearance | Osmotic pressure (300 ± 60 mOsmol/kg) | SDS-PAGE purity (%) | Bio-specific activity value (%) |
|---|---|---|---|---|---|
| | three months | Clear aqueous dosage form, no visible precipitate | 301 | 99 | 110 |
| | four months | Clear aqueous dosage form, no visible precipitate | 294 | - | - |
| | five months | Clear aqueous dosage form, no visible precipitate | 296 | - | - |
| | six months | Clear aqueous dosage form, no visible precipitate | 292 | 97 | 95 |
| Formulation formula 6 | 0 day | Clear aqueous dosage form, no visible precipitate | 290 | 100 | 102 |
| | one month | Clear aqueous dosage form, no visible precipitate | 289 | 105 | 110 |
| | two months | Clear aqueous dosage form, no visible precipitate | 293 | - | - |
| | three months | Clear aqueous dosage form, no visible precipitate | 292 | 98 | 99 |
| | Four months | Clear aqueous dosage form, no visible precipitate | 298 | - | - |
| | five months | Clear aqueous dosage form, no visible precipitate | 300 | - | - |
| | six months | Clear aqueous dosage form, no visible precipitate | 292 | 102 | 112 |

Table -8

In accelerated stability observation for formulation formulas 4-6 at 25 °C, it can be determined that the formulation formulas all met the requirements of a temperature sensitive gel formulation (see Table 8).

**Example 4**

**Eye irritation test with recombinant human serum albumin/epidermal growth factor fusion protein temperature sensitive gel eye drops being repeatedly administered to New Zealand rabbits twice a day for 28 days**

This test was completed by JOINN Laboratories (China) Co., Ltd. Eight male New Zealand rabbits were randomly divided into two groups with four rabbits in each group. Formulation formula 5 was used. Eye drops were administered to the conjunctiva sacs twice a day with an interval of 10-12 hours for 28 consecutive days, according to low-dose group (200 μg/ml) and high-dose group (500 μg/ml). The day of first administration was counted as D1, and during administration (D1-D28), general clinical observation was performed once a day. At D1, D4, D8, D11, D14, D21 and D28, the animals were weighed once. Slit-lamp examination was performed on the animals within 24 hours prior to the start of the test, prior to the first administration at D1, D4, D8, D11, D14, D21 and D28, and 1 h, 2 h, 4 h, 24 h, 48 h and 72 h after the last administration at D28. At D32, all the animals were handed over to a veterinarian for unified management.
During the test, no animal died and no abnormal clinical reaction related to the test article was found. During the test, the integral mean values of eye irritation reaction of two doses of test articles were both 0. According to the evaluation standard of eye irritation, the eye irritation reaction classification of each group at each time point was evaluated as non-irritating. The corneal fluorescence staining of the animals was all negative, and no corneal epithelium damage was found. The results show that the innovative temperature sensitive eye drop formulation formula containing recombinant human serum albumin/epidermal growth factor fusion protein formed by the present disclosure has no visible clinical irritation reaction to rabbit eyes.

**Example 5**

**Skin irritation and sensitization test of innovative temperature sensitive gel eye drop formulation formula containing recombinant human serum albumin/epidermal growth factor fusion protein on epidermis of healthy volunteers**

The skin irritation and sensitization test was carried out by continuously applying a temperature sensitive gel external spray prepared from the formula 5 to the epidermis of the skin on the inside of the arm above the palm of the subject for 7 days. The skin application site was observed for redness, eruptions, blistering and the like at the application site immediately after the application, and the skin at the application site was observed for redness, eruptions, blistering and the like continuously on a daily basis, and each administration process was recorded by recording video or taking photos through mobile phones. The method is to take photos of the local administration site on the skin epidermis before and after administration, or record video during administration.

FIG. 2 shows that recombinant human serum albumin/epidermal growth factor fusion protein temperature sensitive gel eye drops and recombinant human serum albumin/epidermal growth factor fusion protein eye drops (spray) are simultaneously applied to healthy volunteers (left: 0 min after administration; right: 15 min after administration; the upper two figures are from male volunteers, and the lower two figures are from female volunteers), no epidermal irritation, allergy, or damage was found after single or multiple times of application, the temperature sensitive gel eye drops form a gel due to the epidermal temperature after contacting the skin, which can last for at least 15 min; whereas the non-temperature-sensitive non-gel-like eye drops (or sprays) do not aggregate and stay on the skin surface and leave no trace after absorption.

The results show that, after the innovative temperature sensitive formulation formula containing recombinant human serum albumin/epidermal growth factor fusion protein prepared by the present disclosure, whether in the form of eye drops, temperature-sensitive eye drops, external sprays or temperature-sensitive external formulation, has no epidermal irritation reaction, no allergic reaction, no sensitization reaction and no damage after being applied to the human body surface, as shown in FIG. 2: recombinant human serum albumin/epidermal growth factor fusion protein temperature sensitive gel eye drops and recombinant human serum albumin/epidermal growth factor fusion protein eye drops (spray) are simultaneously applied to healthy volunteers (left: 0 min after administration; right: 15 min after administration; the upper two figures are from male volunteers, and the lower two figures are from female volunteers), no epidermal irritation, allergy, or damage was found after single or multiple times of application, the temperature sensitive gel eye drops form a gel due to the epidermal temperature after contacting the skin, which can last for at least 15 min; whereas the non-temperature-sensitive non-gel-like eye drops (or sprays) do not aggregate and stay on the skin surface and leave no trace after absorption.

**Example 6**

**Observation of the repair effect of recombinant human serum albumin/epidermal growth factor fusion protein temperature sensitive gel eye drops on corneal epithelium damage in New Zealand rabbits**

This test was completed by JOINN Laboratories (China) Co., Ltd. 24 male New Zealand rabbits are randomly divided into 4 groups with 6 rabbits in each group. A cornea damage model was established by scraping all corneal epithelium in the right eyes of all animals. After modeling is finished, the cornea was fluorescently stained. Except the animals in the model control group were given normal saline, the other animals were given 50 μL of test article/control article by eye drops every day, the test article (test article 1 was an eye drop formulation, formulation formula 1; test article 2 was temperature sensitive gel eye drops, formulation formula 5) was administered twice a day, while positive control (recombinant human epidermal growth factor eye drop (yeast), Guilin Pavay Gene Pharmaceutical Co., Ltd., batch number: 201709133C) was administered 4 times a day for 7 consecutive days. Fluorescence staining was carried out at 0 h immediately after modeling, and at 24 h, 48 h, 72 h, 96 h, 120 h, 144 h and 168 h after the first administration at different time nodes, corneal pictures were observed and collected by a slit-lamp microscope, the area (mm$^2$) of corneal fluorescence staining was measured, and the area differences between different groups were compared. After modeling, whether the modeling is successful or not can be judged by a fluorescence staining method, so that the damaged area of cornea can be stained, the undamaged area of cornea is not stained, and the repaired area of cornea is not stained, which can be evaluated as successful corneal repair and has curative effect. An area/part of the cornea that is not fluorescently stained indicates that it has been repaired.

Results are shown in FIG. 3: pharmacodynamic study of blank control (normal saline), positive control (rhEGF eye drops commodity) and rHSA/EGF temperature sensitive gel eye drops in a cornea damage animal model established by scraping all cornea epithelium in the right eyes of rabbits. Immediately after modeling, and at 24 h, 48 h, 72 h, 96 h and 120 h after the first administration, corneal pictures were observed and collected by a slit-lamp microscope, the

area (mm$^2$) of corneal fluorescence staining was measured, and the area differences between different groups were compared. The results show that compared with the model control group in the same period, the positive control drug is administered four times a day, and has the curative effect achieved by the temperature-sensitive gel eye drops provided by the present disclosure being administered two times a day, and the curative effect of the temperature-sensitive gel eye drops is not inferior to the positive control drug. For example, compared with the positive control drug, the stained area of corneal fluorescein sodium decreased significantly at 96 hours after the first administration (p≤0.05).

The innovative temperature sensitive eye drop formulation formula containing recombinant human serum albumin/epidermal growth factor fusion protein formed by the present disclosure was confirmed to have repairing effects on the cornea and conjunctiva, and long-lasting remarkable effects of drugs for clinical treatment.

**Example 7**

**Tissue distribution study and pharmacokinetic observation of $^{89}$Zr-recombinant human serum albumin/epidermal growth factor fusion protein temperature sensitive gel eye drops being administered to New Zealand Rabbits for a single time**

This study was carried out and completed by Mitro (Nanjing) Biotech Co., Ltd. New Zealand White rabbits was given $^{89}$Zr-recombinant human serum albumin/epidermal growth factor fusion protein (rHSA/EGF, code: X012) (hereinafter referred to as $^{89}$Zr-X012) by eye drops for a single time, and then subjected to PET/CT scanning to delineate the eyeball, aqueous humor, vitreous body and other regions of interest to obtain radioactive uptake values to study the tissue distribution characteristics of the test drugs in New Zealand White rabbits. Pharmacokinetic studies such as tissue distribution of $^{89}$Zr-X012 administered to New Zealand white rabbits by eye drops for a single time using a $^{89}$Zr isotope labeling tracing method. According to the test, [$^{89}$Zr] labeling was first carried out on the test substance, and animal test was carried out after quality control is qualified. Referring to the [$^{89}$Zr] labeling method, samples were labeled with [$^{nat}$Zr] and then mailed to the client for performing the bioactivity assay of the labeled samples. Three New Zealand white rabbits (with 2 male rabbits and 1 female rabbit) were given $^{89}$Zr-X012 (about 20 μg/eye, about 30 μCi/eye) by eye drops for a single time, and G1-M-01-r was subjected to PET/CT scanning at 0 h (immediately after administration), 2 h, 6 h, 12 h, 24 h, 48 h, 96 h and 168 h after being given $^{89}$Zr-X012; G1-M-02-r and G1-F-01-r were subjected to PET/CT scanning at 0 h (immediately after administration), 2 h, 6 h, 12 h and 24 h after being given $^{89}$Zr-X012, the animals were kept immobile, and CT scanning was completed before/after the PET scanning. Image reconstruction was carried out after scanning was finished, the images and data were processed using PMOD software to delineate the eyeball, aqueous humor, vitreous body and other regions of interest, to obtain the radioactive activity concentration of the region of interest (i.e., the radioactive activity value per unit volume), the standard uptake value (SUV for short) for each viscera was calculated based on the dosage of administration, and the radioactive activity concentration of the test substance was calculated based on the specific activity of the test substance.

$$\text{Corrected activity} = \text{initial activity} \times \frac{(\text{correction time point} - \text{initial time point})}{\text{physical half-life of nuclide}} \quad \text{Formula I}$$

$$\text{Percent injected dose rate per gram of tissue (\% ID/g)} = \frac{\text{radioactive activity concentration of region of interest (μCi/g)}}{\text{dosage of administration (μCi)}} \times 100\% \quad \text{Formula II}$$

$$\text{Standard uptake value} = \frac{\text{radioactive activity concentration of region of interest (μCi/g)}}{\text{dosage of administration (μCi)/weight (g)}} \quad \text{Formula III}$$

$$\text{Radioactivity concentration of tissue (μg Equ/g)} = \frac{\text{radioactive activity concentration of region of interest (μCi/g)}}{\text{specific activity (μCi/μg)}} \quad \text{Formula IV}$$

The pharmacokinetic parameters of the drug in each tissue were calculated using the DAS 3.2. 8 non-compartmental model based on the tissue radioactivity material concentration of each animal. A logarithmic curve was made with radioactivity scanning values local to the organ of interest on the ordinate and PET-CT scanning time on the abscissa, and pharmacokinetic parameters and half-life were calculated according to abscissa time points corresponding to radioactivity decay using PMOD software.

About 1 mL of blood was collected through the ear vein at 0 h, 2 h, 6 h, 12 h, 24 h, 48 h, 96 h and 168 h after the animal code G1-M-01-r was given $^{89}$Zr-X012; blood (approximately 1 mL) was collected via ear vein at 0 h, 2 h, 6 h, 12 h and 24 h after animal codes G1-M-02-r and G1-F-01-r were given $^{89}$Zr-X012, and blood collection was immediately carried out after the PET/CT scanning at each time point finished. At the time points of 0 h, 2 h, 6 h, 12 h, 24 h and 48 h, a pipette was used to transfer 100 μL of whole blood to the radioimmunoassay tube, and the radioactivity was detected by a γ counter; the remaining whole blood was centrifuged at 4 °C and 3000 rpm for 10 min to separate serum. At the time points of 96 h and 168 h, a pipette was used to transfer 100μL of whole blood to the radioimmunoassay tube, the radioactivity was detected by a γ counter, and the whole blood was used to prepare serum. Ten half-lives of $^{89}$Zr nuclides were stored at -80 °C (calculated from the time of administration of experimental animals), and subjected to cold-chain transportation at -20 °C to Tianjin SinoBiotech Ltd. for ELISA detection. The test results show that:

1. Labeling results: X012 can achieve [$^{89}$Zr] labeling, quality control after labeling was qualified, and radiochemical purity (hereinafter referred to as RCP) was all greater than 90%; $^{89}$Zr-X012 with the batch number L19092802 had an RCP of 96.42% after stored at 28 °C for 5 h, and an in vitro stability met the test requirements; the biological activity of the test substance after [$^{nat}$Zr] labeling was 87.3% before labeling, and the activity met the test requirements (70%-130%). When the biological activity was measured, the radioactivity of the sample was already at a safe radioactivity value (after 15 days) before it can be used for cell biological activity measurement, whereby both the radioactively labeling process and the process of waiting for the sample to decay theoretically result in some loss of biological activity occurring, but did not affect the animal test results (see FIG. 4: Radio-HPLC chromatogram of $^{89}$Zr-X012 (batch number L19092802)).

2. Tissue distribution test results: radioactivity was predominantly distributed at the administration site in New Zealand white rabbits being given $^{89}$Zr-X012 by eye drops for a single time. There is some distribution of radioactivity in the eyeball, vitreous body, aqueous humor, stomach, spleen and intestine, and the radioactive concentration in other tissues is very low. The radioactivity of each tissue/body fluid was ranked according to $AUC_{(0-24 h)}$ from large to small as follows: administration site > aqueous humor > stomach > eyeball > intestine > spleen > vitreous body > kidney > brain > liver > heart > tibia > muscle > lung > bone joint. The radioactivity of each tissue/body fluid was ranked according to $AUC_{(0-168 h)}$ from large to small as follows: administration site > aqueous humor > stomach > intestine > spleen > eyeball > vitreous body> brain > kidney > liver > tibia > muscle > heart > lung > bone joint (see FIG. 5, which is a distribution diagram of radioactive exposure in tissues of New Zealand white rabbits 0-24 h after the New Zealand white rabbits are given $^{89}$Zr-X012 by eye drops for a single time).

FIG. 6 is a distribution diagram of radioactive counts for each tissue, showing the metabolic profile of the test drug eye drops being administered to eyeballs of the white rabbits. At 0 h after administration, radioactivity peaked in the administration site, eyeball, vitreous body and aqueous humor with radioactivity concentrations of 17377.1 ng Equ./mL, 900.7 ng Equ./mL, 15.6 ng Equ./mL, and 1390.7 ng Equ./mL, respectively. At 2 h after administration, radioactivity peaked in the stomach with a radioactivity concentration of 138.3 ng Equ./mL, and at 24 h after administration, radioactivity peaked in the spleen and intestine with radioactivity concentrations of 16.6 ng Equ./mL and 16.6 ng Equ./mL, respectively. By 24 h after administration, the radioactivity concentrations in the administration site, eyeball, vitreous body and aqueous humor had decreased to less than 1/10 of the peak value; by 168 h after administration, the radioactivity concentrations in the stomach, spleen and intestine had decreased to less than 1/10 of the peak value, and the radioactivity uptake in other tissues was all very low during the test.

3. Results of whole blood radioactivity detection: after the New Zealand white rabbits were given $^{89}$Zr-X012 by eye drops for a single time, at the time points of 0 h, 2 h, 6 h, 12 h, 24 h and 48 h, a pipette was used to transfer 100 μL of whole blood to the radioimmunoassay tube, and the radioactivity was detected by a γ counter, and was all below the lower detection limit of the γ counter. For 50μl administered for a single eye in a single time, 10 μg of rHSA/EGF was contained, the radioactivity was still not detectable in the blood as detected by a radioactivity method.

**Test conclusion:** the radioactivity was predominantly distributed at the administration site in New Zealand white rabbits being given $^{89}$Zr-X012 by eye drops for a single time. There is some distribution of radioactivity in the eyeball, vitreous body, aqueous humor, stomach, spleen and intestine, and the radioactive concentration in other tissues is very low.

$^{89}$Zr-X012 forms a gel on the surface of the eyeball by administration by eye drops. Small amounts of radioactivity can be distributed through the nasolacrimal duct to the esophagus, stomach and intestine, and trace amounts of radioactivity are absorbed into the blood in the intestine and distributed with the blood circulation to other tissues such as spleen and kidney.

The radioactivity of each tissue/body fluid was ranked according to $AUC_{(0-24 h)}$ from large to small as follows: administration site > aqueous humor > stomach > eyeball > intestine > spleen > vitreous body> kidney > brain > liver > heart > tibia > muscle > lung > bone joint. The radioactivity of each tissue/body fluid was ranked by $AUC_{(0-168 h)}$ from large to small as follows: administration site > aqueous humor > stomach > intestine > spleen > eyeball > vitreous body> brain > kidney > liver > tibia > muscle > heart > lung > bone joint.

Systemic protein equivalents of the animals reached the highest value at 0 h after administration, and decreased obviously to different degrees at 24 h after administration. Systemic protein equivalents of the G1-M-01-r animals had decreased to about 1/3 of the peak value by 168 h after administration.

At 0-24 h, the mean drug half-life for the eyeball of each animal was 53.259 h, and the mean drug half-life for the aqueous humor was 31.729 h.

The above description of the examples is only used to help understand the method of the present disclosure and its core idea. It should be pointed out that, for a person having ordinary skill in the art, several improvements and modifications can also be made to the present disclosure without departing from the principle of the present disclosure, and these improvements and modifications also fall within the protection scope of the claims of the present disclosure. Various modifications to these examples will be obvious to those skilled in the art, and the generic principles defined herein may be implemented in other examples without departing from the spirit or scope of the present disclosure. Thus, the present disclosure is not intended to be limited to the examples shown herein but is to be accorded the widest scope consistent with the principles and novel features disclosed herein.

## Claims

1. A temperature sensitive gel damage repair formulation, comprising components at the following concentrations: 0.01-5 mg/mL recombinant human serum albumin/epidermal growth factor fusion protein, 1.9-2.6% m/m glycine, 3.9-6.2% m/m poloxamer 188, 16.3-18.2% m/m poloxamer 407, and 0.5-100 mM phosphate buffer PB.

2. The temperature sensitive gel damage repair formulation according to claim 1, comprising components at the following concentrations: 0.01-5 mg/mL recombinant human serum albumin/epidermal growth factor fusion protein, 1.9-2.1% m/m glycine, 5.8-6.2% m/m poloxamer 188, 16.3-16.7% m/m poloxamer 407, and 0.5-100 mM PB.

3. The temperature sensitive gel damage repair formulation according to claim 2, comprising components at the following concentrations: 0.2 mg/mL recombinant human serum albumin/epidermal growth factor fusion protein, 2.0% m/m glycine, 6.0% m/m poloxamer 188, 16.5% m/m poloxamer 407, and 10 mM PB.

4. The temperature sensitive gel damage repair formulation according to claim 1, comprising components at the following concentrations: 0.01-5 mg/mL recombinant human serum albumin/epidermal growth factor fusion protein, 1.8-2.2% m/m glycine, 5.8-6.2% m/m poloxamer 188, 17.8-18.2% m/m poloxamer 407, and 0.5-100 mM PB.

5. The temperature sensitive gel damage repair formulation according to claim 1, comprising components at the following concentrations: 0.2 mg/mL recombinant human serum albumin/epidermal growth factor fusion protein, 2.0% m/m glycine, 6.0% m/m poloxamer 188, 18% m/m poloxamer 407, and 10 mM PB.

6. The temperature sensitive gel damage repair formulation according to claim 1, comprising components at the following concentrations: 0.01-5 mg/mL recombinant human serum albumin/epidermal growth factor fusion protein, 2.0-2.5% m/m glycine, 4.9-5.1% m/m poloxamer 188, 17.8-18.2% m/m poloxamer 407, and 0.5-100 mM PB.

7. The temperature sensitive gel damage repair formulation according to claim 6, comprising components at the following concentrations: 0.2 mg/mL recombinant human serum albumin/epidermal growth factor fusion protein, 2.0% m/m glycine, 5.0% m/m poloxamer 188, 18% m/m poloxamer 407, and 10 mM PB.

8. The temperature sensitive gel damage repair formulation according to any one of claims 1 to 7, comprising temperature sensitive gel eye drops and a temperature sensitive gel external spray.

9. An application of the temperature sensitive gel damage repair formulation according to any one of claims 1-8 in preparing a drug for repairing cornea and/or conjunctiva damage.

10. An application of the temperature sensitive gel damage repair formulation according to any one of claims 1-8 in preparing a drug for preventing and/or treating keratitis.

11. An application of the temperature sensitive gel damage repair formulation according to any one of claims 1-8 in preparing a drug for preventing, alleviating or treating keratoconjunctivitis sicca.

**12.** An application of the temperature sensitive gel damage repair formulation according to any one of claims 1-8 in preparing a drug for treating skin epidermal trauma, abrasions or ulcers.

**13.** An application of the temperature sensitive gel damage repair formulation according to any one of claims 1-8 for repairing cornea and/or conjunctiva damage.

**14.** An application of the temperature sensitive gel damage repair formulation according to any one of claims 1-8 in treating keratitis and/or keratoconjunctivitis sicca.

**15.** An application of the temperature sensitive gel damage repair formulation according to any one of claims 1-8 in treating skin epidermal trauma, abrasions or ulcers.

FIG. 1

FIG. 2

Blank control group: normal saline; administration frequency: 4 times a day

Positive control drug: rhEGF eye drops (yeast, Guilin Pavay), administration frequency: 4 times a day,

batch number:201709133C

Test drug: rHSA/EGF eye drops (yeast); administration frequency: twice a day, batch number:X012/1802A

FIG. 3

UV

DAD-280nm Results

| No. | RT | Height | Area | Area% | Resolution | Plates | Asymmetry |
|---|---|---|---|---|---|---|---|
| 1 | 7.845 | 1498 | 52169 | 4.98 | 0.00 | 992 | 0.00 |
| 2 | 8.858 | 31835 | 987329 | 94.34 | 1.17 | 2351 | 1.69 |
| 3 | 11.43 7 | 234 | 7034 | 0.67 | 2.58 | 1304 | 0.00 |
| Totals | | 33567 | 1046532 | 100.00 | | | |

RCP

FC Results

| No. | RT | Height | Area | Area% | Resolution | Plates | Asymmetry |
|---|---|---|---|---|---|---|---|
| 1 | 7.966 | 51842 | 1767282 | 4.15 | 0.00 | 1068 | 0.00 |
| 2 | 8.933 | 1275841 | 40819020 | 95.85 | 1.11 | 2209 | 1.69 |
| Totals | | 1327683 | 42586302 | 100.00 | | | |

FIG. 4

0h      2h      6h      12h      24h    SUV 0-70

48h      96h      168h    SUV 0-70

FIG. 5

FIG. 6

## INTERNATIONAL SEARCH REPORT

| | International application No. |
|---|---|
| | **PCT/CN2021/107365** |

**A.    CLASSIFICATION OF SUBJECT MATTER**

A61K 38/38(2006.01)i；  A61K 9/06(2006.01)i；  A61K 9/08(2006.01)i；  A61P 27/02(2006.01)i；  A61P 17/02(2006.01)i

According to International Patent Classification (IPC) or to both national classification and IPC

**B.    FIELDS SEARCHED**

Minimum documentation searched (classification system followed by classification symbols)

A61K,A61P

Documentation searched other than minimum documentation to the extent that such documents are included in the fields searched

Electronic data base consulted during the international search (name of data base and, where practicable, search terms used)

CNABS, CNMED, DWPI, WPABS, CNKI, ENTXT, CNTXT, ENTXTC, ISI Web of Knowledge, GENBANK, EMBL: 人血清白蛋白, 表皮生长因子, 融合蛋白, 甘氨酸, 泊洛沙姆, PB缓冲液, 凝胶, 温度敏感, 修复制剂, HAS, GF, EGF, epidiuen cell growth, fusion protein, glycin, plolxamer, eye, gel, PB buffer slution

**C.    DOCUMENTS CONSIDERED TO BE RELEVANT**

| Category* | Citation of document, with indication, where appropriate, of the relevant passages | Relevant to claim No. |
|---|---|---|
| PX | CN 113244380 A (FORTUNEROCK BIOTECHNOLOGY (BEIJING) CO., LTD.; TIANJIN SINOBIOTECH LTD.; BEIJING MEIFUYUAN BIOMEDICAL TECHNOLOGY CO., LTD.; TIANJIN LINDA BIOTECHNOLOGY CO., LTD.) 13 August 2021 (2021-08-13) entire document | 1-15 |
| Y | CN 102311503 A (TIANJIN SINO BIO-TECHNOLOGY CO.,LTD.; FORTUNEROCK, INC.) 11 January 2012 (2012-01-11) claims 1-10 | 1-15 |
| Y | CN 101972224 A (STAIDSON (BEIJING) BIOPHARMACEUTICALS CO., LTD.) 16 February 2011 (2011-02-16) claims 1-14 | 1-15 |
| A | WO 2007036107 A1 (GENESCIENCE PHARMACEUTICALS CO. et al.) 05 April 2007 (2007-04-05) entire document | 1-15 |
| A | US 2006195945 A1 (LI SUN et al.) 31 August 2006 (2006-08-31) entire document | 1-15 |

☐ Further documents are listed in the continuation of Box C.     ☑ See patent family annex.

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **14 March 2022** | **01 April 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

International application No.

**PCT/CN2021/107365**

| Patent document cited in search report | | | Publication date (day/month/year) | Patent family member(s) | | | Publication date (day/month/year) |
|---|---|---|---|---|---|---|---|
| CN | 113244380 | A | 13 August 2021 | None | | | |
| CN | 102311503 | A | 11 January 2012 | None | | | |
| CN | 101972224 | A | 16 February 2011 | CN | 101972224 | B | 04 January 2012 |
| WO | 2007036107 | A1 | 05 April 2007 | EP | 1941901 | A1 | 09 July 2008 |
| | | | | EP | 1941901 | A4 | 05 August 2009 |
| | | | | EP | 1941901 | B1 | 03 April 2013 |
| | | | | US | 2009156482 | A1 | 18 June 2009 |
| | | | | US | 7858582 | B2 | 28 December 2010 |
| | | | | CN | 1939534 | A | 04 April 2007 |
| | | | | CN | 1939534 | B | 01 December 2010 |
| US | 2006195945 | A1 | 31 August 2006 | AU | 2003245089 | A1 | 23 January 2004 |
| | | | | KR | 20050028011 | A | 21 March 2005 |
| | | | | KR | 100628024 | B1 | 26 September 2006 |
| | | | | WO | 2004005520 | A1 | 15 January 2004 |
| | | | | AU | 2003245090 | A1 | 23 January 2004 |
| | | | | WO | 2004005340 | A1 | 15 January 2004 |
| | | | | KR | 20050036946 | A | 20 April 2005 |
| | | | | KR | 100711145 | B1 | 24 April 2007 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110722585
- WO ZL200710057571 A
- WO 2009043277 A1
- US 8603973 B2

**Non-patent literature cited in the description**

- *Chinese Medicinal Biotechnology,* 2017
- Chinese Pharmacopoeia. 2015, vol. III